(19) Europäisches Patentamt
European Patent Office
Office européen des brevets

(11) Veröffentlichungsnummer: **0 221 376 B1**

# (12) EUROPÄISCHE PATENTSCHRIFT

(45) Veröffentlichungstag der Patentschrift: **20.06.90**

(51) Int. Cl.[5]: **C07D 401/06**, A61K 31/445

(21) Anmeldenummer: **86113837.8**

(22) Anmeldetag: **06.10.86**

(54) **N-substituierte Diphenylpiperidine.**

(30) Priorität: **07.10.85 US 785113**

(43) Veröffentlichungstag der Anmeldung: **13.05.87 Patentblatt 87/20**

(45) Bekanntmachung des Hinweises auf die Patenterteilung: **20.06.90 Patentblatt 90/25**

(84) Benannte Vertragsstaaten: **AT BE CH DE FR GB IT LI LU NL SE**

(56) Entgegenhaltungen:
**EP-A- 0 183 216**
**FR-A- 2 260 349**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE AG, Postfach 3255, CH-4002 Basel(CH)**

(72) Erfinder: **Mullin, John Guilfoyle, Jr., 519 Goffle Hill Road, Hawthorne, N.J.(US)**
Erfinder: **Kierstead, Richard Wightman, 30 Willowbrook Drive, North Caldwell, N.J.(US)**
Erfinder: **Triscari, Joseph, 98 North Fulton Street, Bloomfield, N.J.(US)**

(74) Vertreter: **Lederer, Franz, Dr. et al, Van der Werth, Lederer & Riederer Patentanwälte Lucile-Grahn-Strasse 22, D-8000 München 80(DE)**

EP 0 221 376 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue N-substituierte Diphenylpiperidine der Formel

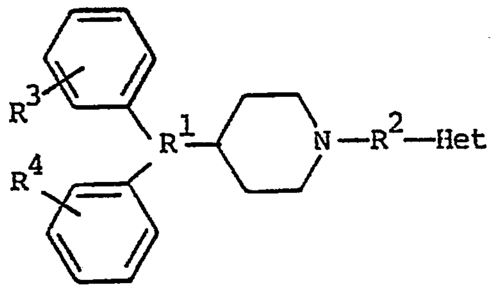

I

worin
$R^1$ CH, C=CH oder CH–$CH_2$
$R^2$ $C_{1-12}$-Alkylen oder CO–$(CH_2)_n$, n 0 bis 11,
$R^3$ und $R^4$ Wasserstoff, Halogen oder $C_{1-7}$-Alkoxy,
Het Pyridyl, Pyrimidinyl oder Imidazolyl sind,
und Salze davon mit pharmazeutisch verwendbaren Säuren.

Die Erfindung betrifft ferner ein Verfahren zur Herstellung dieser Verbindungen, neue in diesem Vefahren vorkommende Zwischenprodukte, sowie Medikamente auf der Basis der besagten Verbindungen.

Im Rahmen der Erfindung bedeutet "nieder-Alkoxy" eine geradkettige oder verzweigte Gruppe mit 1-7 C-Atomen, wie Methoxy, Aethoxy, Propoxy, Isopropoxy, n-Butoxy, t-Butoxy, Pentoxy und Hexyloxy. "Alkylen" bezeichnet vorzugsweise eine geradkettige oder verzweigte Gruppe mit 1-12, insbesondere 4-10 C Atome, wie Methylen, Aethylen, Propylen, Butylen, Pentylen, Hexylen, Heptylen, Octylen, Nonylen, Decylen oder Dodecylen. "Halogen" bezeichnet Chlor, Brom, Jod und Fluor. "Het" bezeichnet vorzugsweise 3-Pyridyl, 5,Pyrimidinyl oder 1-Imidazolyl.

Beispiele von Salzen der Verbindungen der Formel I sind solche mit pharmazeutisch verwendbaren anorganischen Säuren, z.b. Halogenwasserstoffsäure, wie Chlor-, Brom- oder Jodwasserstoffsäure, Schwefelsäure, Phosphorsäure oder Salpetersäure, oder mit organischen Säuren, wei Weinsäure, Zitronensäure, Fumarsäure, Camphersulfonsäure, Methansulfonsäure, Toluolsulfonsäure, Salicylsäure, Ascorbinsäure, Maleinsäure oder Mandelsäure. Bevorzugte Salze sind die Hydrohalogenide, insbesondere die Hydrochloride, und die Maleate, Fumarate und Methansulfonate.

Bevorzugte Verbindungen der Formel I sind diejenigen, worin $R^1$ C=CH und/oder worin $R^2$ $C_{1-12}$-, insbesondere $C_{4-10}$-Alkylen, und/oder worin $R^3$ und $R^4$ Wasserstoff und/oder worin Het Pyridyl sind.

Beispiele von solchen Verbindungen sind:
3-[7-(4-(2,2-Diphenyläthenyl)-1-piperidinyl]heptyl]pyridin (1:1)-(E)-2-butendioat-hemihydrat,
3-[6-[4-(2,2-Diphenyläthenyl)-1-piperidinyl]hexyl]pyridin-(1:1)(E)-2-butendioat,
3-[5-[4-(2,2-Diphenyläthenyl)-1-piperidinyl]pentyl]pyridin (1:1)-(E)-2-butendioat und insbesondere
3-[8-[4-(2,2-Diphenyläthenyl)-1-piperidinyl]-octyl]pyridin und
3-[10-[4-(2,2-Diphenyläthenyl)-1-piperidinyl]decyl]pyridin.

Weitere Beispiele von Verbindungen der Formel I sind:
3-[8-[4-2,2-Bis(3-fluorphenyl)äthenyl]-1-piperidinyl]octyl]pyridin
3-[8-[4-[2,2-Bis(2-fluorphenyl)äthenyl]-1-piperidinyl]octyl]pyridin
1-[6-[4-[2,2-Bis(4-flouorphenyl)äthenyl]-1-piperidinyl]hexyl]-1H-imidazol
3-[8-[4-[2,2-Bis(2-methoxyphenyl)äthenyl]-1-piperidinyl]octyl]pyridin
1-[6-[4-[2,2-Bis(4-methoxyphenyl)äthenyl]-1-piperidinyl]hexyl]-1H-imidazol
5-[6-[4-[2,2-Bis-(3-fluorphenyl)äthenyl]-1-piperidinyl]hexyl]pyrimidin.

Die Verbindungen der Formel I und ihre Salze können dadurch hergestellt werden, dass man eine Verbindung der Formel

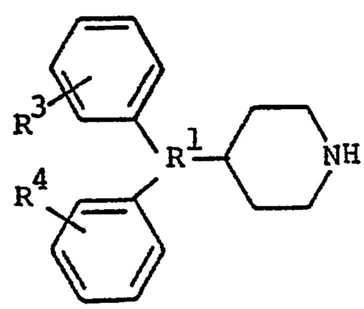

II

mit einem Halogenid der Formel Hal-$R^2$-Het, worin $R^1$ bis $R^4$ und Het die obige Bedeutung haben, umsetzt, gewünschtenfalls, eine erhaltene Verbindung der Formel I, worin $R^2$ eine Carbonylgruppe enthält, zur entsprechenden Verbindung der Formel I, worin $R^2$ Alkylen ist, reduziert und gewünschtenfalls eine erhaltene freie Base der Formel I in Form eines Salzes mit einer pharmazeutisch verwendbaren Säure isoliert.

Die Reaktion einer Verbindung der Formel II mit einem Halogenid Hal-$R^2$-Het kann in an sich bekannter Weise, z.B. in einem Lösungsmittel in Gegenwart einer Base durchgeführt werden. Wenn $R^2$ eine Carbonylgruppe enthält, verwendet man vorzugsweise ein aprotisches Lösungsmittel, z.B. Tetrahydrofuran (THF), Benzol oder Toluol. Als Base verwendet man in diesem Fall, z.B. Pyridin oder Triäthylamin, und die Reaktionstemperatur beträgt zweckmässig etwa 0°C bis Rückflusstemperatur, insbesondere 0°C. Wenn $R^2$ Alkylen ist, verwendet man als Lösungsmittel z.B. Dimethylformamid (DMF), Dimethylsulfoxid oder ein Alkanol, wie Aethanol, und als Base, z.B. wasserfreies Natriumcarbonat, ein Natriumalkoxid, wie Natriummethoxid, oder ein wässriges Alkalimetallhydroxid, wie Natriumhydroxid, und die Reaktionstemperatur beträgt zweckmässig etwa 0°C bis etwa 100°C, insbesondere etwa 50-75°C.

Die fakultative Reduktion einer Verbindung der Formel I kann man in an sich bekannte Weise durchführen, z.B. in einem Lösungsmittel, wie THF oder einem Kohlenwasserstoff, z.B. Toluol oder Benzol, mit einem Hydrid, wie Lithiumaluminiumhydrid oder Natrium-bis(2-methoxyäthoxy)aluminiumhydrid, bei einer Temperatur von etwa 0°C bis Rückflusstemperatur, vorzugsweise bei Raumtemperatur.

Die Salze der Verbindungen der Formel I sind vorzugsweise in einem Lösungsmittel, z.B. Aethanol, Aceton oder Acetonitril, hergestellt, indem man die freie Base mit der entsprechenden wasserfreien Säure umsetzt.

Unter den Verbindungen der Formel II sind diejenigen der Formel

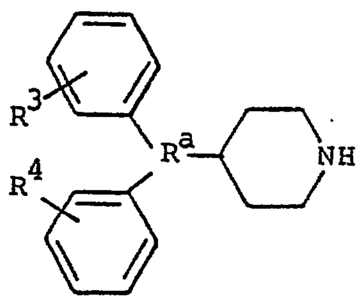

II-a

worin $R^a$ C=CH oder CH=$CH_2$ und $R^3$ und $R^4$ die obige Bedeutung haben,
neu und als solche Bestandteil der Erfindung. Sie können in an sich bekannter Weise durch Hydrierung eines Pyridinderivates der Formel

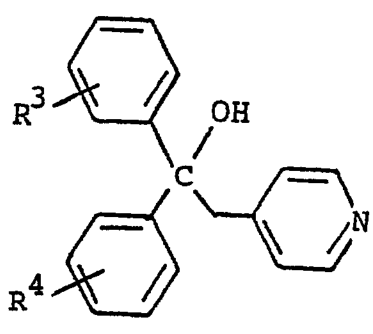

III

worin $R^3$ und $R^4$ obige Bedeutung haben,
zum entsprechenden Piperidinderivat, gefolgt von Dehydratisierung des letzteren zur entsprechenden Verbindung der Formel II-a, worin $R^a$ C=CH ist, und gewünschtenfalls, Hydrierung zur Verbindung der Formel II-a, worin $R^a$ CH–$CH_2$ ist.

Die Hydrierung einer Verbindung der Formel III kann man in an sich bekannter Weise durchführen, z.B. in einem Lösungsmittel, wie Eisessig, in Gegenwart eines Hydrierungskatalysators, z.B. Platinoxid, mit Wasserstoff, z.B. unter einem Druck bis zu etwa 6,8 bar, vorzugsweise etwa 3,4 bar und bei einer Temperatur im Bereich von etwa 0 bis etwa 100°C, vorzugsweise etwa 25-50°C.

Die Dehydratisierung des erhaltenen Piperidinderivates kann man in Gegenwart von Trifluoressigsäure oder mit einer Säure, wie Toluolsulfonsäure, in einem Kohlenwasserstoff, wei Toluol oder Benzol, bei einer Temperatur im Bereich von etwa 0°C bis etwa 50°C, vorzugsweise bei etwa 25°C, bewerkstelligen.

Die fakultative Hydrierung einer Verbindung der Formel II-a kann man in einem Lösungsmittel, z.B. einem Alkanol, wie Aethanol, mittels eines Katalysators wei 10% Pd/C, in Gegenwart von Wasserstoff unter gewöhnlichem Druck und bei einer Temperatur von 25°C durchführen.

Die Verbindungen der Formeln II, worin $R^1$ CH ist, III und Hal-$R^2$-Het sind bekannt oder können nach an sich bekannten Methoden hergestellt werden. So kann man die Halogenide Hal-$R^2$-Het, worin $R^2$ Alkylen ist, durch Halogenierung der entsprechenden Alkohole, z.B. mit Thionylchlorid, in einem Lösungsmittel, wie einem halogenierten Kohlenwasserstoff, z.B. Methylenchorid, oder einem Kohlenwasserstoff, wie Benzol oder Toluol, bei einer Temperatur im Bereich von etwa 0° bis Rückflusstemperatur, vorzugsweise bei Rückflusstemperatur herstellen.

Die Verbindungen der Formel I und ihre pharmazeutisch verwendbaren Säureadditionssalze weisen Insulin-erniedrigende Wirksamkeit auf und können daher als Mittel zur Behandlung von Obesitas Verwendung finden. Diese Wirksamkeit kann man nach an sich bekannten Methoden nachweisen, z.B. wie beschrieben in der US-Patentschrift 4 500 540, durch Vergleich der Insulinsekretion vor und nach Perfusion des isolierten Ratten-Pankreas mit der Testverbindung. Die Resultate sind in der Tabelle I als Hemmungsindex wiedergegeben, wobei ein Hemmungsindex von mehr als 1 die vollständige Hemmung der Insulinsekretion bedeutet.

In der Tabelle I sind auch die Resultate der Glukose-Clearance angegeben. In diesem Test werden die Glukosespiegel einerseits unmittelbar vor der Verabreichung von Glukose an Sprague-Dawley-Ratten und andererseits 30, 60 und 90 Minuten nach Glukoseverabreichung ermittelt. Im Vergleich zu den Kontrollen erhöhen aktive Verbindungen den Glukosespiegel. Da der Glukosespiegel 30 Minuten nach Verabreichung der Glukose einen Maximum erreicht, wird der Unterschied zwischen dem Glukosespiegel bei Kontrollen und bei behandelten Ratten 30 Minuten nach Verabreichung als Index der Aktivität verwendet. Die Resultate sind in % der Kontrollen angegeben, wobei Substanzen mit einem Index von mehr als 100% wirksam sind.

Tabelle I

| Freie Base der Formel I der Beispiele 1-25 | Perfusion des Pankreas Konzentration (mM) | Hemmungs-index | Glukose-Clearance (% der Kontrolle) |
|---|---|---|---|
| 1 | 100 | 6,0 | 105 |
| 2 | 50 | 7,6 | 99 |
| 5 | 100 | 14,1 | 109 |
| 7 | 100 | 5,3 | 120 |
| 8 | 100 | 1,7 | 101 |
| 9 | 100 | 0,6 | 113 |
| 10 | 100 | 24,1 | 97 |
| 11 | 100 | 19,1 | 106 |
| 12 | 100 | 16,3 | 136 |
| 13 | 100 | 10,1 | 101 |
| 14 | 100 | 2,6 | 144 |
| 15 | 50 | 35 | 118 |
| 16 | 50 | 38 | 127 |
| 17 | 50 | 2,0 | 125 |
| 19 | 100 | 3,0 | 147 |
| 20 | 100 | 10,4 | 104 |
| 21 | 50 | 1,4 | 100 |
| 22 | 50 | 2,7 | 107 |
| 23 | 50 | | 100 |
| 24 | 50 | 2,5 | 120 |
| 25 | 50 | 1,1 | 101 |

Die Wirkung der Produkte der Formel I gegen Obesitas wurde an Zuckerratten nach der in der US-Patentschrift 4 500 540 beschriebenen Methode bestimmt. Die Resultate sind in der Tabelle II für die freie Base des Beispiels 16 und in der Tabelle III für die freien Base von weiteren Beispielen angegeben.

Tabelle II

| Dosis (mg/kg) | Körpergewicht-Zunahme (g) | Kadaver-Lipide Unterschied (g) | Futtereinnahme (% der Kontrolle) | Zirkulierendes Insulin (% der Kontrolle) |
|---|---|---|---|---|
| 9 | +9 | | 91 | 83 |
| 17 | -16 | | 89 | 76 |
| 24 | -32 | | 82 | 47 |
| 34 | -55 | | 83 | 33 |
| 28 | -88 | -62 | 81 | 47 |

Tabelle III

| Beispiel | Dosis (mg/kg) | Körpergewicht-Zunahme (g) | Futtereinnahme (% der Kontrolle) | Relatives Körpergewicht / Zunahmeindex |
|---|---|---|---|---|
| 16 | 38 | -49 | 73 | 92 |
| 16 | 58 | -76 | 70 | 94 |
| 21 | 49 | -12 | 100 | 17 |

Man kann die Verbindungen der Formel I als Medikamente, z.B. in Form von pharmazeutischen Präparaten, die sie zusammen mit einem verträglichen organischen oder anorganischen inerten Träger enthalten, verabreichen. Beispiele von solchen Trägern sind Wasser, Gelatine, Laktose, Stärke, Magnesiumstearat, Talk, pflanzliche Oele, Gummi oder Polyalkylenglykole. Die pharmazeutischen Präparate können für die enterale, z.B. orale oder parenterale Verabreichung geeignet sein und können in fester Form, z.B. als Tabletten, Dragées, Suppositorien oder Kapseln, oder in flüssiger Form, z.B. als Lösungen, Suspensionen oder Emulsionen, vorliegen. Die pharmazeutischen Präparate können sterilisiert sein und/oder Hilfsstoffe wie Konservierungs-, Stabilisierungs-, Netz- oder Emulgiermittel, Salze zum Erzeugen eines osmotischen Druckes oder Puffer enthalten. Sie können auch weitere therapeutisch wirksame Substanzen enthalten.

Die pharmazeutischen Dosierungsmengen können etwa 250 bis 500 mg einer Verbindung der Formel I oder eines pharmazeutischen verwendbaren Säureadditionssalzes davon enthalten. Orale Dosierungsmengen können etwa 5 bis etwa 30 mg/kg/Tag betragen.

Die folgenden Beispiele veranschaulichen die Erfindung.

A. Herstellung der Alkohole Het–$R^2$–OH

a) Ein Gemisch von 6,40 l 1,3-Diaminopropan und 15 g Lithiumdraht wurde im Ölbad auf 65°C erhitzt. Zusätzliche 153 g Lithiumdraht wurden portionenweise zugesetzt. Der auf 20°C abgekühlten Lösung wurden 1,56 kg Kalium-t-butoxid zugesetzt. Die Lösung wurde 15 Minuten bei 20°C gerührt. 500 g 3-Octin-1-ol in 100 ml THF wurden zugesetzt. Das Gemisch wurde über Nacht bei 20 °C gerührt und dann unter Rühren in 12 l Eiswasser geschüttet. Es wurden 8 l Eis zugesetzt und das Gemisch wurde mit dreimal 8 l Methylenchlorid extrahiert. Die vereinigten Extrakte wurden mit 8 l Wasser, 8 l 1N Salzsäure und 4 l Kochsalzlösung gewaschen und dann getrocknet. Nach Entfernung des Lösungsmittels und Destillation erhielt man 338,2 g (68% Ausbeute) 7-Octin-1-ol in Form einer farblosen Flüssigkeit, Sdp. 104°C/16 mbar.

b) Ein Gemisch von 34,6 g 7-Octin-1-ol, 26,4 ml Brompyridin, 115 ml Triäthylamin und 350 ml Dichlormethan wurde unter Argonstrom gerührt. Dem Gemisch wurden 3,85 g Bis-triphenylphosphin-palladium(II)-chlorid und 0,365 g Kupfer(I)jodid zugesetzt und dann wurde das Gemisch über Nacht unter Rückfluß erhitzt. Das abgekühlte Reaktionsgemisch wurde mit Äther verdünnt und die Feststoffe wurden filtriert und mit Äther gewaschen. Die Filtrate wurden mehrmals mit 1N Salzsäure extrahiert und die vereinigten wäßrigen Schichten wurden mit Überschuß Natriumhydroxid stark basisch gestellt. Das Gemisch wurde mit Dichlormethan extrahiert, getrocknet und eingedampft, wobei man 51 g (91%) rohes 8-(3-Pyridyl)-7-octin-1-ol erhielt. Durch katalytische Hydrierung in 1000 ml Äthanol mittels 5 g 10% Pd/C erhielt man nach Destillation 36 g (72%) 3-Pyridinoctanol, Sdp. 145–152°C/0,4 mbar.

| | | | | |
|---|---|---|---|---|
| Analyse, | berechnet für $C_{13}H_{21}NO$: | C, 75,32; | H, 10,21; | N, 6,76; |
| | gefunden: | C, 75,13; | H, 10,21; | N, 6,66. |

c) Durch Ersatz von 7-Octin-1-ol durch 3-Butin-1-ol und 5-Hexin-1-ol erhielt man:
1) 5-Pyrimidinbutanol, Sdp. 175–180°C/0,4 mbar

| | | | | |
|---|---|---|---|---|
| Analyse, | berechnet für $C_8H_{12}N_2O$: | C, 63,13; | H, 7,95; | N, 18,41; |
| | gefunden: | C, 62,83; | H, 7,98; | N, 18,63, bzw. |

2) 5-Pyrimidinhexanol, Sdp. 170–175°C/0,8 mbar

| | | | | |
|---|---|---|---|---|
| Analyse, | berechnet für $C_{10}H_{16}N_2O$: | C, 66,63; | H, 8,95; | N, 15,54; |
| | gefunden: | C, 66,62; | H, 9,15; | N, 15,59. |

B. Herstellung der Halogenide Hal–$R^2$–Het ($R^2$ = Alkylen)

Einer Lösung von 0,03 Mol des Alkohols von A. in 40 ml Methylenchlorid wurde tropfenweise eine Lösung von 0,04 Mol Thionylchlorid in 10 ml Methylenchlorid zugesetzt. Die entstandene Lösung wurde 1 Stunde unter Rückfluß erhitzt, dann abgekühlt und mit gesättigter Kaliumcarbonatlösung gewaschen. Die organischen Schichten wurden getrocknet und bei 25°C eingedampft. Das entstandene Öl wurde ohne weitere Reinigung in der nächsten Stufe verwendet.

Auf diese Weise erhielt man aus 145,8 g 3-Pyridinoctanol 191,3 g 3-(8-Chloroctyl)pyridin in Form eines Öls.

C. Herstellung der Halogenide Hal–$R^2$–Het [$R^2$ = CO–$(CH_2)_n$]

In Eis abgekühltem Thionylchlorid (20 ml) wurden feste Pyridincarbonsäure zugesetzt. Das Gemisch wurde 15 Minuten bei 25°C gerührt und dann aus Toluol mehrmals zur Trockene eingedampft. Das entstandene rohe Säurechlorid-hydrochlorid wurde ohne weitere Reinigung in der nächsten Stufe verwendet.

D. Herstellung der Verbindungen der Formel III

a) Eine Lösung von 252,5 g 4-Picolin und 2,50 l THF wurde unterhalb von 20°C abgekühlt und bei 15–20°C mit 1,315 l einer Lösung von 1,85 M Phenyllithium in 70:30 Cyclohexan:Äther versetzt. Das Gemisch wurde 30 Minuten bei 15–20°C gerührt und dann auf –5–0°C abgekühlt. Bei dieser Temperatur wurde eine Lösung von 494,2 g Benzophenon in 1,50 l THF zugesetzt. Das Gemisch wurde dann bei Raumtemperatur 3 Stunden gerührt. Nach Zusatz von 1,0 l deionisiertem Wasser und 2,0 l THF wurde das Gemisch zur Auflösung der Feststoffe erwärmt. Die wäßrige Phase wurde abgetrennt und mit 2,0 l THF extrahiert. Die vereinigten organischen Lösungen wurden getrocknet. Das Lösungsmittel wurde abgedampft. Der resultierende Feststoff wurde bei Rückflußtemperatur mit 1,60 l Hexan vermischt. Die Suspension lwurde über Nacht bei 4°C aufbewahrt und dann filtriert. Die Feststoffe wurden mit 1,0 l Petroläther gewaschen und getrocknet, wobei man 699,6 g (94% Ausbeute) α,α-Diphenyl-4-pyridinäthanol, Smp. 154–155°C erhielt.

b) In ähnlicher Weise wurde α,α-Bis(3-methoxyphenyl)-4-pyridinäthanol, Smp. 134–135°C hergestellt.

E. Hydrierung der Verbindungen der Formel III

a) 40 g α,α-Diphenyl-4-pyridinäthanol wurden unter 3,4 bar in 500 ml Eisessig mittels 4,0 g Platinoxyd hydriert. Nach Filtrierung und Abdampfen wurde der feste Rückstand in einem heissem Gemisch von Wasser und Toluol gelöst. Ein Ueberschuss an Natriumhydroxidlösung wurde dann zugesetzt und das entstandene Gemisch wurde abgekühlt und filtriert. Das rohe Produkt wurde aus Toluol umkristallisiert, wobei man 32,5 g (80%) α,α-Diphenyl-4-piperidinäthanol, Smp. 153-154°C erhielt. Das Hydrochlorid wurde aus Methanol kristallisiert, Smp. 271°C (Zers.).

b) Analog erhielt man aus α,α-Bis(4-fluorphenyl)-4-pyridinäthanol α,α-Bis(4-fluorphenyl)-4-piperidinäthanol, Smp. 161-163°C.

c) In ähnlicher Weise wurde eine Lösung von 63 g α,α-Bis(3-methoxyphenyl)-4-pyridinäthanol in 400 ml Essigsäure mittels 2 g Platinoxid zu 62,5 g α,α-Bis(3-Methoxyphenyl)-4-piperidinäthanol, Smp. 111-113°C hydriert.

d) In einer Variante des Verfahrens a) wurde 260 g α,α-Diphenyl-4-pyridinäthanol in 3,0 l Aethanol zu 190,65 g (72%) α,α-Diphenyl-4-piperidinäthanol in Gegenwart von 26 g 5% Rh/C als Katalysator hydriert.

F. Herstellung der Verbindungen der Formel II-a

a) 20 g α,α-Diphenyl-4-piperidinäthanol wurden in 40 ml wasserfreiem Trifluoressigsäure gelöst und 15 Minuten gerührt. Leicht siedende Stoffe wurden unter Vakuum entfernt und der Rückstand wurde zwischen Toluol und 2N Natriumhydroxidlösung aufgetrennt. Die organischen Schichten wurden getrocknet und eingedampft und der Rückstand wurde aus Hexan zu 17,2 g (92%) 4-(2,2-Diphenyläthenyl)piperidin, Smp. 98-99°C kristallisiert.

b) Analog wurden aus 25 g α,α-Bis(4-fluorphenyl)-4-piperidinäthanol, 0.25 molarem Methylbenzensolvat und 50 ml Trifluoressigsäure, 19.6 g (83%) des Hexansolvats des 4-[2,2-Bis(4-fluorphenyl)-äthenyl]-piperidin, Smp. 84-86°C erhalten.

c) Analog wurde eine Lösung von 15 g α,α-Bis(3-methoxyphenyl)-4-piperidinäthanol in 30 ml Trifluoressigsäure in 4-[2,2-Bis(3-methoxyphenyl)äthenyl]piperidin, Smp. des Hydrobromid 135,5-137°C umgewandelt.

d) 15,2 g 4-(2,2-Diphenyläthenyl)piperidin in 500 ml Aethanol wurden mittels 1,5 g 10% Pd/C hydriert. Nach Filtrieren und Eindampfen erstarrte das resultierende Oel in ein tiefsiedendes Wachs. Das 4-(2,2-Diphenyläthyl)-piperidin-hydrochlorid, Smp. 178-179°C wurde aus 2-Propanol/Aether umkristallisiert.

e) In ähnlicher Weise erhielt man 4-[2,2-Bis(4-fluorphenyl)äthyl]piperidin-hydrochlorid als farbloses Oel.

G. Herstellung der Verbindungen der Formel I

Beispiel 1

Ein Gemisch von 2,0 g 4-Diphenylmethylpiperidin in 5 ml DMF und 4,2 ml Triäthylamin wurde bei 0°C mit einer Lösung von 5 ml DMF und dem aus 2,0 g 3-Pyridinbuttersäure erhaltenem Säurechlorid behandelt. Nach 15 Minuten wurde das Gemisch mit Chlormethan verdünnt, filtriert und zur Trockene eingedampft. Der Rückstand wurde zwischen Toluol und 1N Natriumhydroxid verteilt und die organische Schicht wurde mit Kochsalzlösung gewaschen, getrocknet und eingedampft, wobei man 3,3 g rohes Oel erhielt. Dieses wurde durch Chromatographie auf Silicagel mittels 98:2 Aethylacetat/Triäthylamin gereinigt. Das erhaltene farblose Oel wurde unter Vakuum bei 70°C getrocknet wobei man 2,8 g (86%) 4-Diphenylmethyl-l-[1-oxo-4-(3-pyridyl)butyl]piperidin, 0,1 molares Aethylacetatsolvat erhielt.

| | | | | |
|---|---|---|---|---|
| Analyse, berechnet für | $C_{27}H_{30}N_2O,0,lC_4H_8O_2$: | C, 80,79; | H, 7,62; | N, 6,88; |
| | gefunden: | C, 80,91; | H, 7,57; | N, 6,81. |

Beispiel 2

Analog Beispiel 1 erhielt man aus 7,9 g 4-(2,2,-Diphenyläthenyl)-piperidin und dem aus 6,6 g 3-Pyridinbuttersäure hergestelltem Säurechlorid, nach Kristallisieren der Toluolextrakte aus Dichlormethan/Aether, 9,1 g (74%) 4-(2,2-Diphenyläthenyl-1-[1-oxo-4-(3-pyridyl)butyl]piperidin, Smp. 91-93°C

Beispiel 3

Analog Beispiel 1 erhielt man aus 9,0 g 4-[2,2-Bis(4-fluorphenyl)äthenyl]piperidin, 0,15 molarem Hexansolvat und dem aus 6,6 g 3-Pyridinbuttersäure erhaltenem Säure-chlorid, nach Kristallisieren des Toluolextraktes aus Aether, 8,7 g (65%) 4-[2,2-bis[4-fluorphenyl)äthenyl]1-[1-oxo-4-(3-pyridyl)butyl]-piperidin, Smp. 100-102°C.

Beispiel 4

Analog Beispiel 1 erhielt man aus 5,27 g 4-(2,2-Diphenyläthenyl)piperidin und dem ausgehend von 6,5 g 3-Pyridinpentansäure erhaltenen Säurechlorid, 8,0 g (94%) rohes 4-(2,2-Diphenyläthenyl)-1-[1-oxo-5-(3-pyridyl)pentyl] piperidin, Smp. 82-84°C nach Kristallisieren aus Aether.

Beispiel 5

Analog Beispiel 1 erhielt man aus 5,3 g 4-(2,2-Diphenyläthyl)piperidin und dem aus 5,0 g 3-Pyridinbuttersäure erhaltenen Säurechlorid 6,3 g (76%) 4-(2,2-Diphenyläthyl)-1-[1-oxo-4-(3-pyridyl)butyl]-piperidin, Smp. 98-100°C nach Kristallisieren aus Methylenchlorid/Aether.

Beispiel 6

Analog Beispiel 1 erhielt man aus 8,3 g 4-[2,2-Bis(4-fluorphenyl)äthyl]piperidin und dem aus 6,1 g 3-Pyridinbuttersäure erhaltenen Säurechlorid 10,5 g (85%) 4-[2,2-Bis(4-fluorphenyl)äthyl]-1-[1-oxo -4-(3-pyridyl)butyl]piperidin, Smp. 108-110°C nach Kristallisieren aus Methylenchlorid/Aether.

Beispiel 7

Eine Lösung von 7,8 g des Produktes von Beispiel 1 in 100 ml Toluol wurde unter Rühren tropfenweise mit 12 ml einer 3,5M Lösung von Natrium-bis(2-methoxyäthoxy)aluminiumhydrid in Toluol behandelt. Nach 30 Minuten wurde das Gemisch tropfenweise mit einem Ueberschuss an Natriumhydroxid behandelt und mit Toluol extrahiert. Die organischen Schichten wurden mit Kochsalzlösung gewaschen, getrocknet und eingedampft, wobei man 6,9 g (90%) rohe freie Base in Form eines Oels erhielt. Eine Lösung von 5,9 g dieser Base in 50 ml heissem Aethanol wurde mit 1,8 g Maleinsäure behandelt und die Lösung wurde dann mit 150 ml Aether verdünnt. Der entstandene Feststoff wurde wie oben beschrieben umkristallisiert und man erhielt 6,7 g (87%) 3-[4-[4-(Diphenylmethyl)-1-piperidinyl]butyl]pyridin -(1:1)-(Z)-2-butendioat, Smp. 140-141°C.

Beispiel 8

Analog Beispiel 7 wurde aus 5,0 g des Produktes von Beispiel 2 und 7,2 ml einer 3,5M Lösung von Natriumbis(2-methoxyäthoxy)aluminiumhydrid in Toluol, 4,4 g 3-[4-[4-(2,2-Diphenyläthenyl)-1-piperidinyl]-butyl]pyridin, Smp. 54-46°C erhalten. Diese wurden in 50 ml heissem Aethanol gelöst und mit 1,28 g Fumarsäure behandelt. Nach Verdünnung mit 50 ml Aether erhielt man 4,1 g (67%) Fumarat, Smp. 163-165°C.

Beispiel 9

Analog Beispiel 7 erhielt man aus 13,0 g des Produktes von Beispiel 3 und 34 ml einer 3,4M Lösung von Natrium-bis(2-methoxyäthoxy)aluminiumhydrid in Toluol, 8,9 g freie Base als Oel. Eine Lösung der Base in 50 ml heissem Methanol wurde mit 2,3 g Fumarsäure behandelt und das Methanol wurde abdestilliert unter Zusatz von Aethylacetat. Die entstandenen Feststoffe wurden aus Methanol/Aethylacetat umkristallisiert und man erhielt 8,9 g (56%) reines 3-[4-[4-[2,2-Bis(4-fluorphenyl)äthenyl]-1-piperidinyl]butyl] pyridin-(1:1)-(E)-2-butendioat, Smp. 183-184°C (Zersetzung).

Beispiel 10

Analog Beispiel 7 erhielt man aus 7,4 g des Produktes von Beispiel 4 und 10,0 ml einer 3,5M Lösung von Natrium-bis(2-methoxyäthoxy)aluminiumhydrid in Toluol, 7,4 g freie Base in Form eines Oels. Die freie Base wurde wie in Beispiel 9 behandelt und man erhielt 8,4 g (92%) 3-[5-[4-(2,2-Diphenyläthenyl)-1-piperidinyl]pentyl]pyridin -(1:1)-(E)-2-butendioat, Smp. 160-161°C(Zersetzung).

Beispiel 11

Analog Beispiel 7 erhielt man aus 6,1 g des Produktes von Beispiel 5 und 9 ml einer 3,5M Lösung von Natrium-bis(2-methoxyäthoxy)aluminiumhydrid in Toluol, 5,4 g (90%) der freien Base 3-[4-[4-(2-2-Diphenyläthyl)-1-piperidinyl]-butyl]pyridin vom Smp. 62-64°C nach Kristallisieren aus Hexan. Eine Lösung von 5,25 g der freien Base und 1,53 g Fumarsäure in 30 ml heissem Methanol wurde mit 50 ml Aether verdünnt und abgekühlt. Man erhielt 6,4 g (96%) des Fumarats, Smp. 146-148°C.

Beispiel 12

Analog Beispiel 7 analoger Weise erhielt man aus 11,6 g des Produktes von Beispiel 6 und 30 ml einer 3,4M Lösung von Natrium-bis(2-methoxyäthoxy)aluminiumhydrid, 7,4 g (65%) freie Base in Form eines Oels. Eine Lösung von 7,4 g der freien Base und 2,0 g Fumarsäure in 50 ml Methanol wurde mit Aethylacetat verdünnt und abgekühlt. Nach Filtrierung und Umkristallisation aus THF/Aether erhielt man 7,1 g (76%) 3-[4-[4-[2,2-Bis(4-fluorphenyl)äthyl]-1-piperidinyl]butyl]pyridin-(1:1)-(E)-2-butendioat, Smp. 155-156°C (Zersetzung).

Beispiel 13

Ein Gemisch von 4,0 g 4-(2,2-Diphenyläthenyl)piperidinyl, 4,64 g 3-(3-Brompropyl)-pyridin-hydrobromid, 8,25 ml 4N Natriumhydroxid und 50 ml DMF wurde 3 Stunden bei 25°C gerührt. Tiefsiedende Stoffe wurden entfernt und der Rückstand wurde zwischen Aethylacetat und 1N Natriumhydroxid verteilt. Die organischen Schichten wurden getrocknet und eingedampft und der Rückstand (5,8 g) wurde auf Silicagel mittels 96:2:2 Aethylacetat-Methanol-Triäthylamin chromatographiert. Die erhaltenen Fraktionen wurden vereinigt und eingedampft wobei man 4,9 g (85%) freie Base in Form eines Oels erhielt. Eine Lösung von 4,9 g der freien Base in Methanol wurde wie in Beispiel 9 behandelt und man erhielt 4,3 g (66%) 3-[3-[4-(2,2-Diphenyläthenyl) -1-piperidinyl]-propyl]pryidin-(1:1)-(E)-2-butendioat, Smp. 154-156°C (Zersetzung).

Beispiel 14

Ein Gemisch von 4,64 g 4-(2,2-Diphenyläthenyl)piperidin, 3,7 g 3-(6-Chlorhexyl)pyridin, 2,64 g Natriumjodid und 50 ml DMF wurde 18 Stunden bei 50°C gerührt. Das Lösungsmittel wurde abgedampft und der Rückstand wurde zwischen 1N Natriumhydroxid und Aethylacetat verteilt. Das entstandene Oel (7,4 g) wurde auf Silicagel mittels 98:2 Aethylacetat-Triäthylamin chromatographiert. Nach Eindampfen der erhaltenen Fraktionen erhielt man 3,9 g (52%) freie Base in Form eines Oels. Eine Lösung von 3,9 g der freien Base in 25 ml heissem Methanol wurde mit 1,07 g Fumarsäure wie in Beispiel 9 behandelt und man erhielt 4,0 g (82%) 3-[6-[4-(2,2-Diphenyläthenyl)-1-piperidinyl]hexyl]pyridin -(1:1)-(E)-2-butendioat, Smp. 170-172°C (Zersetzung).

Beispiel 15

Analog Beispiel 14 erhielt man aus 3,9 g 4-(2,2-Diphenyläthenyl)piperidin und 3,1 g 3-(7-Chlorheptyl)pyridin, 4,0 g (62%) freie Base in Form eines Oels. Nach Kristallisation des Fumarats aus Aethanol/Aethylacetat erhielt man reines 3-[7-[4-(2,2-Diphenyläthenyl) -1-piperidinyl]heptyl]pyridin -(1:1)-(E)-2-butendioat-hemihydrat, Smp. 96-99°C (Zersetzung).

Beispiel 16

Analog Beispiel 14 erhielt man aus 8,64 g 4-(2,2-Diphenyläthenyl)piperidin und 7,4 g 3-(8-Chloroctyl)pyridin, 11,0 g (74%) 3-[8-[4-(2,2-Diphenyläthenyl) -1-piperidinyl]octyl]pyridin, Smp. 54-55°C nach Umkristallisation aus Hexan. Das Fumarat wurde aus Aethanol/Aceton kristallisiert, Smp 128-129°C.

Beispiel 17

Ein Gemisch von 7,4 g 3-(6-Chlorhexyl)pyridin, 10,3 g 4-[2,2-Bis(4-fluorphenyl)äthyl]piperidin, 5,6 g Natriumjodid und 100 ml DMF wurde 18 Stunden bei 50°C und 1 Stunde bei 100°C gerührt. Das Lösungsmittel wurde abgedampft und der Rückstand wurde zwischen Aethylacetat und 1N Natriumhydroxid verteilt. Die organischen Schichten wurden getrocknet und eingedampft und das Produkt (10,5 g) wurde auf Silicagel mittels 50:50:2 Hexan/Aethylacetat/Triäthylamin chromatographiert. Die erhaltenen Fraktionen wurden vereinigt und eingedampft, wobei man 8,5 g (53%) freie Base in Form eines Oels erhielt. Eine Lösung von 8,5 g der freien Base in Methanol wurde wie in Beispiel 9 behandelt und man erhielt 7,5 g (72%) 3-[6-[4-[2,2-Bis(4-fluorphenyl)äthyl] -1-piperidinyl]hexyl]pyridin-(1:1)-(E)-2-butendioat, Smp. 140-141°C.

Beispiel 18

7,3 g 3-[8-[4-(2-2-Diphenyläthenyl)-1-piperidinyl]octyl]pyridin wurde unter normalen Bedingungen mittels 100 ml Aethanol und 1,5 g 10% Pd/C als Katalysator hydriert. Nach Filtrierung und Eindampfen wurde der Rückstand aus Hexan kristallisiert und man erhielt 6,0 g (82%) 3-[8-[4-(2,2-Diphenyläthyl) -1-piperidinyl]octyl]pyridin, Smp. 68-70°C. Das Fumarat wurde aus Aceton kristallisiert, Smp. 110-111°C.

Beispiel 19

Analog Beispiel 14 erhielt man aus 4,64 g 4-(2,2-Diphenyläthenyl)piperidin und 3,0 g 5-(4-Chlorbutyl)pyrimidin (hergestellt aus 5-Pyrimidinbutanol) 3,6 g (52%) freie Base in Form eines Oels. Nach Kristallisieren des Fumarats aus Methanol/Aethylacetat erhielt man 3,0 g 5-[4-[4-(2,2-Diphenyläthenyl)-1-piperidinyl]butyl]pyrimidin(1:1)-(E)-2-butendioat, Smp. 181-182°C.

Beispiel 20

Analog Beispiel 14 erhielt man aus 7,4 g 4-(2,2-Diphenyläthenyl)piperidin und 5,9 g 5-(6-Chlorhexyl)pyrimidin, 5,9 g (50%) 5-[6-[4-(2,2-Diphenyläthenyl)-1-piperidinyl]hexyl]pyrimidin, Smp. 88-90°C nach Kristallisation aus Hexan.

Beispiel 21

Analog Beispiel 1 erhielt man aus 7,9 g 4-(2,2-Diphenyläthenyl)piperidin und dem aus 8,24 g 3-Pyridinnonansäure hergestellten Säurechlorid 12,4 g (73,7%) 4-(2,2-Diphenyläthenyl) -1-[1-oxo-9-(3-pyridyl)nonyl]piperidin, Smp. 82-83°C nach Kristallisation aus Aether.

Beispiel 22

Analog Beispiel 7 erhielt man aus 6,5 g des Produktes von Beispiel 21 und 7,7 ml einer 3,5M Lösung von Natrium-bis(2-methoxyäthoxy)aluminiumhydrid in Toluol, 3-[9-[4-2(2,2-Diphenyläthenyl)-1-piperidinyl]-nonyl]pyridin, Smp. 51-53°C nach Kristallisieren aus Hexan. Nach Eindampfen der Hexanfiltrate erhielt man 5,9 g eines Produktes, das nach Auflösung in 25 ml heissem Methanol mit 1,47 g Fumarsäure behandelt wurde. Das Methanol wurde abdestilliert unter Zusatz von Aceton und die entstandenen Feststoffe wurden aus Methanol/Aceton umkristallisiert. Man erhielt 6,7 g (85%) 3-[9-[4-(2,2-Diphenyläthenyl)-1-piperidinyl]nonyl] pyridin-fumarat, Smp. 125-127°C.

Beispiel 23

Analog Beispiel 14 erhielt man aus 6,59 g 4-(2,2-Diphenyläthenyl)piperidin und 6,4 g 3-(10-Chlordecyl)pyridin 10,4 g (86%) 3-[10-[4-(2,2-Diphenyläthenyl)-1-piperidinyl]deoyl]pyridin, Smp. 63-65°C nach Kristallisation aus Hexan. Das Fumarat wurde aus Aceton kristallisiert, Smp. 134-136°C.

Beispiel 24

Analog Beispiel 1 erhielt man aus 3,4 g 4-(2,2-Diphenyläthenyl)piperidin und dem aus 2,73 g 6-(1H-imidazol-1-yl)hexansäure hergestellten Säurechlorid 4,7 g (84%) 4-(2,2-Diphenyläthenyl)-1-[1-oxo-6-(1H-imidazol-1-yl)hexyl]piperidin, Smp. 98-100°C nach Kristallisation aus Aethylacetat/Hexan.

Beispiel 25

In zu Beispiel 7 analoger Weise erhielt man aus 2,5 g des 4-(2,2-Diphenyläthenyl) -1-[2-oxo-6-(1H-imidazol-1-yl)hexyl]-piperidin und 3,4 ml einer 3,5M Lösung von Natrium-bis(2-methoxyäthoxy)aluminiumhydrid in Toluol 1,7 g (70%) 1-[6-[4-(2,2-Diphenyläthenyl)-1-piperidinyl]hexyl]-1H-imidazol, Smp. 104-106°C nach Kristallisieren aus Aether/Hexan.

Beispiel 26

Ein Gemisch von 1,65 g 4-[2,2-bis(3-Methoxyphenyl)-äthenyl]piperidin, 1,13 g 3-(8-Chloroctyl)pyridin, 0,75 g Natriumjodid und 0,53 g wasserfreiem Natriumcarbonat in 10 ml DMF wurde 16 Stunden bei 75°C unter Argon gerührt. Das Lösungsmsittel wurde im Vakuum entfernt und der Rückstand wurde in einem Gemisch von Aethylacetat und einer 1N Natriumhydroxidlösung aufgenommen. Die abgetrennten wässrigen Schichten wurden mit 3 Portionen Aethylacetat erneut extrahiert. Die organischen Schichten wurden mit Wasser und Kochsalzlösung gewaschen, dann vereinigt, getrocknet, mit Aktivkohle entfärbt und eingedampft, wobei man 2,5 g eines Oels erhielt. Dieses wurde durch HPLC (Hexan:Aethylacetat:Triäthylamin, 34:17:1) gereinigt und man erhielt 1,8 g 3-[8-[4-[2,2-Bis(3-methoxyphenyl)-äthenyl] -1-piperidinyl]octyl]pyridin in Form eines Oels.

Eine Lösung von 1,7 g der erhaltenen Base in Aethanol wurde mit 418 mg Oxalsäuredihydrat behandelt. Dann wurde zur Auflösung der Feststoffe unter Rückfluss erhitzt, filtriert und mit 30 ml Aether verdünnt. Das Gemisch wurde abgekühlt und der entstandene Feststoff wurde filtriert. Man erhielt 2,0 g 3-[8-[4-[2,2-Bis(3-methoxyphenyl)äthenyl] -1-piperidinyl]octyl]pyridin-(1:1)-äthandioat, 0,25 molar Hydrat, Smp. 112-115°C.

Beispiel 27

In an sich bekannter Weise wurden Tabletten und Kapseln folgender Zusammensetzung hergestellt:

| A. | Bestandteil: | mg/Tablette: | |
|---|---|---|---|
| | 3-[8-[4-(2,2-Diphenyläthenyl)-1-piperidinyl]octyl]pyridin | 250 | 500 |
| | Laktose | 100 | 200 |
| | Polyvinylpyrrolidon | 10 | 20 |
| | Modifizierte Stärke | 10 | 20 |
| | Magnesiumstearat | 3 | 6 |

| B. | Bestandteil: | mg/Tablette: | |
|---|---|---|---|
| | 3-[8-[4-(2,2-Diphenyläthenyl)-1-piperidinyl]octyl]pyridin | 250 | 500 |
| | Laktose | 75 | 150 |
| | Pregelatinisierte Stärke | 15 | 30 |
| | Mikrokristalline Cellulose | 75 | 150 |
| | Magnesiumstearat | 3 | 6 |

| B. | Bestandteil: | mg/Kapsel: | |
|---|---|---|---|
| | 3-[8-[4-(2,2-Diphenyläthenyl)-1-piperidinyl]octyl]pyridin | 250 | 500 |
| | Pregelatinisierte Maisstärke | 20 | 40 |
| | Modifizierte Stärke | 10 | 20 |
| | Talk | 10 | 20 |
| | Magnesiumstearat | 1 | 2 |

**Patentansprüche für die Vertragsstaaten: BE, CH, DE, FR, GB, IT, LI, LU, NL, SE**

1. N-substituierte Diphenylpiperidine der Formel

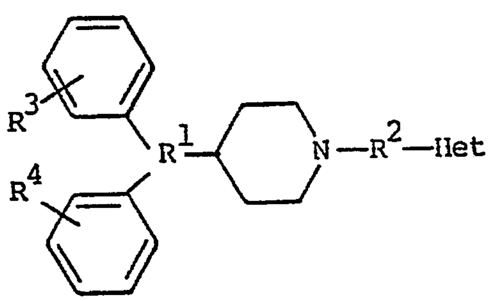

I

worin
$R^1$ CH, C=CH oder CH–$CH_2$
$R^2$ $C_{1-12}$-Alkylen oder CO–$(CH_2)_n$, n 0 bis 11,
$R^3$ und $R^4$ Wasserstoff, Halogen oder $C_{1-7}$-Alkoxy,
Het Pyridyl, Pyrimidinyl oder Imidazolyl sind,
und Salze davon mit pharmazeutisch verwendbaren Säuren.

2. Verbindungen nach Anspruch 1, worin $R^1$ C=CH ist.

3. Verbindungen nach Anspruch 1 oder 2, worin $R^2$ $C_{1-12}$, insbesondere $C_{4-10}$-Alkylen ist.

4. Verbindungen nach Anspruch 1, 2 oder 3, worin $R^3$ und $R^4$ Wasserstoff sind.

5. Verbindungen nach Anspruch 1, 2, 3 oder 4, worin Het Pyridyl ist.

6. Eine Verbindung aus der Gruppe der folgenden:
3-[7-[4-(2,2-Diphenyläthenyl)-1-piperidinyl]heptyl]-pyridin-(1:1)-(E)-2-butendioat-hemihydrat,
3-[6-[4-(2,2-Diphenyläthenyl)-1-piperidinyl]hexyl]-pyridin-(1:1)-(E)-2-butendioat,
3-[5-[4-(2,2-Diphenyläthenyl)-1-piperidinyl]pentyl]-pyridin-(1:1)-(E)-2-butendioat und insbesondere
3-[8-[4-(2,2-Diphenyläthenyl)-1-piperidinyl]-octyl]pyridin und
3-[10-[4-(2,2-Diphenyläthenyl)-1-piperidinyl]decyl]pyridin.

7. N-unsubstituierte Diphenylpiperidine der Formel

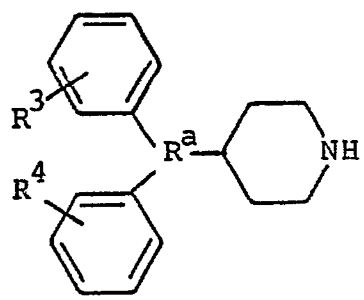

II-a

worin $R^a$ C=H oder $CH{=}CH_2$ und $R^3$ und $R^4$ wie in Anspruch 1 sind.

8. Eine Verbindung nach einem der Ansprüche 1-6 zur Verwendung als therapeutisch aktive Substanz, insbesondere zur Senkung des Insulinspiegels und zur Behandlung von Obesitas.

9. Verfahren zur Herstellung von Verbindungen nach einem der Ansprüche 1-6, dadurch gekennzeichnet, dass man eine Verbindung der Formel

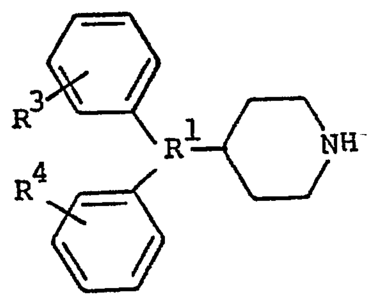

II

mit einem Halogenid der Formel Hal-$R^2$-Het, worin $R^1$ bis $R^4$ und Het wie im Anspruch 1 sind, umsetzt, gewünschtenfalls, eine erhaltene Verbindung der Formel I, wiron $R^2$ eine Carbonylgruppe enthält, zur entsprechenden Verbindung der Formel I, worin $R^2$ $C_{1-12}$-Alkylen ist, reduziert und gewünschtenfalls eine erhaltene freie Base der Formel I in Form eines Salzes mit einer pharmazeutisch verwendbaren Säure isoliert.

10. Pharmazeutisches Präparat, insbesondere zur Behandlung von Obesitas, auf der Basis einer Verbindung nach einem der Ansprüche 1–6.

11. Verwendung einer Verbindung nach einem der Ansprüche 1–6 bei der Herstellung eines Präparats nach Anspruch 10.

**Patentansprüche für den Vertragsstaat: AT**

1. Verfahren zur Herstellung von N-substituierten Diphenylpiperidinen der Formel

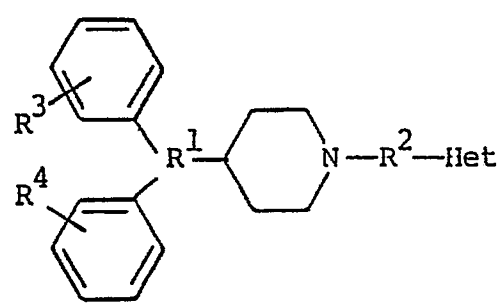

I

worin
$R^1$ CH, C=CH oder CH–$CH_2$
$R^2$ $C_{1-12}$-Alkylen oder CO–$(CH_2)_n$, n 0 bis 11,
$R^3$ und $R^4$ Wasserstoff, Halogen oder $C_{1-7}$-Alkoxy,
Het Pyridyl, Pyrimidinyl oder Imidazolyl sind,
und von Salzen davon mit pharmazeutisch verwendbaren Säuren, dadurch gekennzeichnet, daß man eine Verbindung der Formel

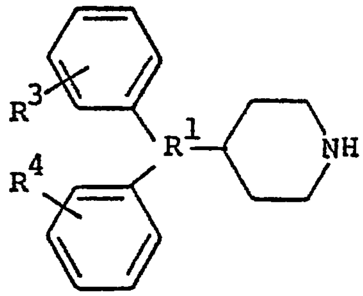

II

mit einem Halogenid der Formel Hal–$R^2$–Het, worin $R^1$ bis $R^4$ und Het obige Bedeutung haben, umsetzt, gewünschtenfalls, eine erhaltene Verbindung der Formel I, worin $R^2$ eine Carbonylgruppe enthält, zur entsprechenden Verbindung der Formel I, worin $R^2$ $C_{1-12}$-Alkylen ist, reduziert und gewünschtenfalls eine erhaltene freie Base der Formel I in Form eines Salzes mit einer pharmazeutisch verwendbaren Säure isoliert.

2. Verfahren nach Anspruch 1, worin $R^1$ C=CH ist.
3. Verfahren nach Anspruch 1 oder 2, worin $R^2$ $C_{1-12}$, insbesondere $C_{4-10}$-Alkylen ist.
4. Verfahren nach Anspruch 1, 2 oder 3, worin $R^3$ und $R^4$ Wasserstoff sind.
5. Verfahren nach Anspruch 1, 2, 3 oder 4, worin Het Pyridyl ist.
6. Verfahren nach Anspruch 1, dadurch gekennzeichnet, daß man eine Verbindung aus der Gruppe der folgenden herstellt:
3-[7-[4-(2,2-Diphenyläthenyl)-1-piperidinyl]heptyl]-pyridin-(1:1)-(E)-2-butendioat-hemihydrat,
3-[6-[4-(2,2-Diphenyläthenyl)-1-piperidinyl]hexyl]-pyridin-(1:1)-(E)-2-butendioat,
3-[5-[4-(2,2-Diphenyläthenyl)-1-piperidinyl]pentyl]-pyridin-(1:1)-(E)-2-butendioat und insbesondere
3-[8-[4-(2,2-Diphenyläthenyl)-1-piperidinyl]-octyl]-pyridin und
3-[10-[4-(2,2-Diphenyläthenyl)-1-piperidinyl]decyl]-pyridin.

**Claims for the Contracting States: BE, CH, DE, FR, GB, IT, LI, LU, NL, SE**

1. N-Substituted diphenylpiperidines of the formula

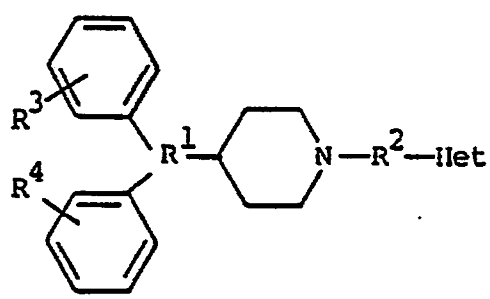

I

wherein
$R^1$ is CH, C=CH or CH–$CH_2$
$R^2$ is $C_{1-12}$-alkylene or CO–$(CH_2)_n$, n is 0 to 11,
$R^3$ and $R^4$ are hydrogen, halogen or $C_{1-7}$-alkoxy,
Het is pyridyl, pyrimidinyl or imidazolyl,
and salts thereof with pharmaceutically usable acids.

2. Compounds according to claim 1, wherein $R^1$ is C=CH.
3. Compounds according to claim 1 or 2, wherein $R^2$ is $C_{1-12}$, especially $C_{4-10}$-alkylene.
4. Compounds according to claim 1, 2 or 3, wherein $R^3$ and $R^4$ are hydrogen.
5. Compounds according to claim 1, 2, 3 or 4, wherein Het is pyridyl.
6. A compound from the following group:
3-[7-[4-(2,2-Diphenylethenyl)-1-piperidinyl]heptyl]-pyridine (1:1)-(E)-2-butenedioate hemihydrate,

3-[6-[4-(2,2-diphenylethenyl)-1-piperidinyl]hexyl]-pyridine (1:1)-(E)-2-butenedioate, 3-[5-[4-(2,2-diphenylethenyl)-1-piperidinyl]pentyl]-pyridine (1:1)-(E)-2-butenedioate and especially 3-[8-[4-(2,2-diphenylethenyl)-1-piperidinyl]-octyl]-pyridine and 3-[10-[4-(2,2-diphenylethenyl)-1-piperidinyl]decyl]-pyridine.

7. N-Unsubstituted diphenylpiperidines of the formula

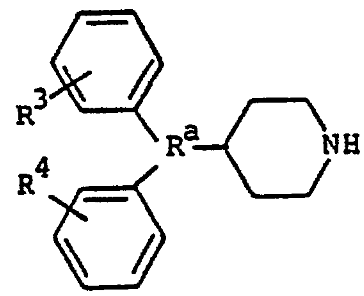

II-a

wherein $R^a$ is C=CH or $CH=CH_2$ and $R^3$ and $R^4$ are as in claim 1.

8. A compound according to any one of claims 1–6 for use as a therapeutically active substance, especially for lowering the insulin level and for treating obesity.

9. A process for the manufacture of compounds according to any one of claims 1–6, characterized by reacting a compound of the formula

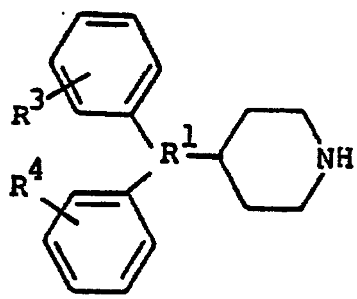

II

with a halogenide of the formula Hal–$R^2$–Het, wherein $R^1$ to $R^4$ and Het are as in claim 1, if desired, reducing an obtained compound of formula I, wherein $R^2$ contains a carbonyl group, to the corresponding compound of formula I, wherein $R^2$ is $C_{1-12}$-alkylene, and, if desired, isolating an obtained free base of formula I in the form of a salt with a pharmaceutically usable acid.

10. A pharmaceutical preparation, especially for the treatment of obesity, based on a compound according to any one of claims 1–6.

11. The use of a compound according to any one of claims 1–6 in the manufacture of a preparation according to claim 10.

**Claims for the Contracting State: AT**

1. A process for the manufacture of N-substituted diphenylpiperidines of the formula

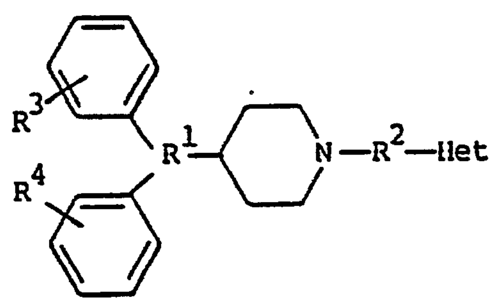

I

wherein
$R^1$ is CH, C=CH or $CH–CH_2$
$R^2$ is $C_{1-12}$-alkylene or $CO–(CH_2)_n$, n is 0 to 11,
$R^3$ and $R^4$ are hydrogen, halogen or $C_{1-7}$-alkoxy,

Het is pyridyl, pyrimidyl or imidazolyl,
and of salts thereof with pharmaceutically usable acids, characterized by reacting a compound of the formula

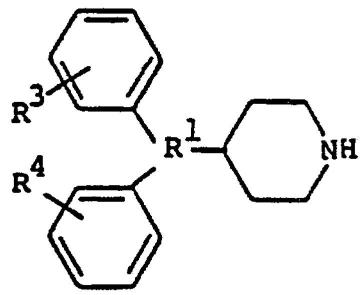

II

with a halogenide of the formula Hal–$R^2$–Het, wherein $R^1$ to $R^4$ and Het have the above significance, if desired, reducing an obtained compound of formula I, wherein $R^2$ contains a carbonyl group, to the corresponding compound of formula I, wherein $R^2$ is $C_{1-12}$-alkylene, and, if desired, isolating an obtained free base of formula I in the form of a salt with a pharmaceutically usable acid.

2. A process according to claim 1, wherein $R^1$ is C=CH.

3. A process according to claim 1 or 2, wherein $R^2$ is $C_{1-12}$, especially $C_{4-10}$-alkylene.

4. A process according to claim 1, 2 or 3, wherein $R^3$ and $R^4$ are hydrogen.

5. A process according to claim 1, 2, 3 or 4, wherein Het is pyridyl.

6. A process according to claim 1, characterized in that a compound from the following group is manufactured:
3-[7-[4-(2,2-Diphenylethenyl)-1-piperidinyl]heptyl]-pyridine (1:1)-(E)-2-butenedioate hemihydrate,
3-[6-[4-(2,2-diphenylethenyl)-1-piperidinyl]hexyl]-pyridine (1:1)-(E)-2-butenedioate,
3-[5-[4-(2,2-diphenylethenyl)-1-piperidinyl]pentyl]-pyridine (1:1)-(E)-2-butenedioate and especially
3-[8-[4-(2,2-diphenylethenyl)-1-piperidinyl]-octyl]-pyridine and
3-[10-[4-(2,2-diphenylethenyl)-1-piperidinyl]decyl]-pyridine.

**Revendications pour les Etats contractants: BE, CH, DE, FR, GB, IT, LI, LU, NL, SE**

1. Diphénylpipéridines substituées à l'azote, de formule

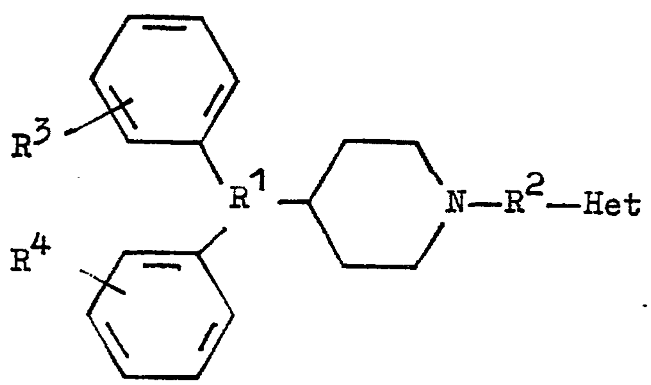

I

dans laquelle
$R^1$ représente un groupe CH, C=CH ou CH–$CH_2$,
$R^2$ représente un groupe alkylène en $C_1$–$C_{12}$ ou CO–$(CH_2)_n$, n a une valeur de 0 à 11,
$R^3$ et $R^4$ représentent l'hydrogène, des halogènes ou des groupes alcoxy en $C_1$–$C_7$,
Het représente un groupe pyridyle, pyrimidinyle ou imidazolyle, et les sels de ces composés et d'acides acceptables pour l'usage pharmaceutique.

2. Composés selon la revendication 1, dans lesquels $R^1$ représente C=CH.

3. Composés selon la revendication 1 ou 2, dans lesquels $R^2$ représente un groupe alkylène en $C_1$–$C_{12}$, plus spécialement en $C_4$–$C_{10}$.

4. Composés selon la revendication 1, 2 ou 3, dans lesquels $R^3$ et $R^4$ représentent l'hydrogène.

5. Composés selon la revendication 1, 2, 3 ou 4, dans lesquels Het représente un groupe pyridyle.

6. Un composé du groupe des suivants:
le (E)-2-butène-dioate de 3-[7-[4-(2,2-diphényléthényl)-1-pipéridinyl]-heptyl]-pyridine (1:1) à l'état d'hémihydrate,

le (E)-2-butène-dioate de 3-[6-[4-(2,2-diphényléthényl)-1-pipéridinyl]-hexyl]-pyridine (1:1),
le (E)-2-butène-dioate de 3-[5-[4-(2,2-diphényléthényl)-1-pipéridinyl]-pentyl]-pyridine (1:1), et plus spécialement
la 3-[8-[4-(2,2-diphényléthényl)-1-pipéridinyl]-octyl]-pyridine, et
la 3-[10-[4-(2,2-diphényléthényl)-1-pipéridinyl]-décyl]-pyridine.

7. Diphénylpipéridines non substituées à l'azote, de formule

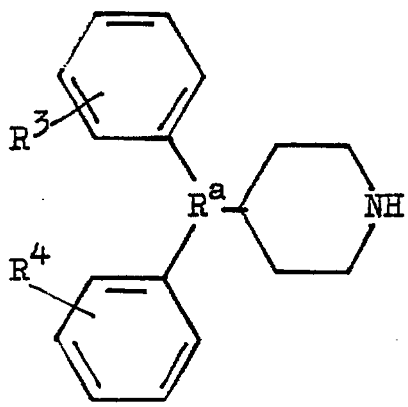

II-a

dans laquelle $R^a$ représente C=CH ou $CH{=}CH_2$ et $R^3$ et $R^4$ ont les significations indiquées dans la revendication 1.

8. Un composé selon l'une des revendications 1 à 6, pour l'utilisation en tant que substance active thérapeutique, en particulier pour le traitement de l'excès d'insuline et de l'obésité.

9. Procédé de préparation des composés selon l'une des revendications 1 à 6, caractérisé en ce que l'on fait réagir un composé de formule

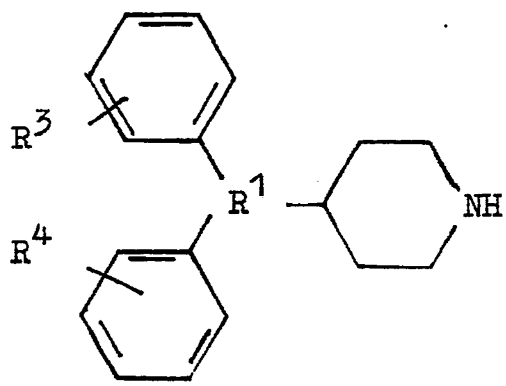

II

avec un halogénure de formule $Hal{-}R^2{-}Het$ dans laquelle les symboles $R^1$ à $R^4$ et Het ont les significations indiquées dans la revendication 1, et si on le désire, on réduit un composé obtenu, répondant à la formule I dans laquelle $R^2$ représente un groupe carbonyle, en le composé correspondant de formule I dans laquelle $R^2$ représente un groupe alkylène en $C_1$–$C_{12}$, et si on le désire, on isole une base libre de formule I ainsi obtenue à l'état de sel d'acide acceptable pour l'usage pharmaceutique.

10. Composition pharmaceutique, prévue en particulier pour le traitement de l'obésité, à base d'un composé selon l'une des revendications 1 à 6.

11. Utilisation d'un composé selon l'une des revendications 1 à 6 pour la préparation d'une composition selon la revendication 10.

**Revendications pour l'Etat contractant: AT**

1. Procédé de préparation de diphénylpipéridines substituées à l'azote et répondant à la formule

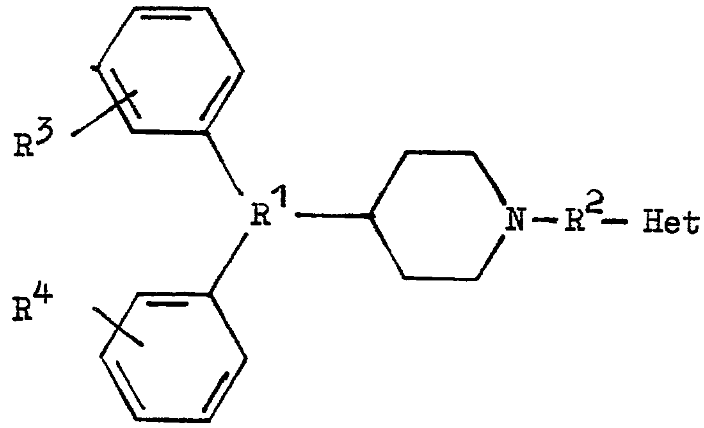

I

dans laquelle
$R^1$ représente un groupe CH, C=CH ou CH–$CH_2$,
$R^2$ représente un groupe alkylène en $C_1$–$C_{12}$ ou CO–$(CH_2)_n$, n a une valeur de 0 à 11,
$R^3$ et $R^4$ représentent l'hydrogène, des halogènes ou des groupes alcoxy en $C_1$–$C_7$,
Het représente un groupe pyridyle, pyrimidinyle ou imidazolyle, et des sels de ces composés et d'acides acceptables pour l'usage pharmaceutique, caractérisé en ce que l'on fait réagir un composé de formule

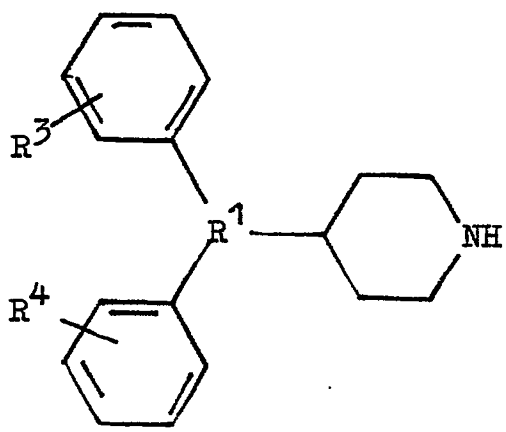

II

aveo un halogénure de formule Hal–$R^2$–Het, dans lesquels les symboles $R^1$ à $R^4$ et Het ont les significations indiquées ci-dessus, et si on le désire, on réduit un composé obtenu, répondant à la formule I dans laquelle $R^2$ représente un groupe carbonyle, en le composé correspondant de formule I dans laquelle $R^2$ représente un groupe alkylène en $C_1$–$C_{12}$, et si on le désire, on isole une base libre de formule I ainsi obtenue à l'état de sel d'acide acceptable pour l'usage pharmaceutique.

2. Procédé selon la revendication 1, dans laquelle $R^1$ représente C=CH.

3. Procédé selon la revendication 1 ou 2, dans lequel $R^2$ représente un groupe alkylène en $C_1$–$C_{12}$, plus spécialement en $C_4$–$C_{10}$.

4. Procédé selon la revendication 1, 2 ou 3, dans lequel $R^3$ et $R^4$ représentent l'hydrogène.

5. Procédé selon la revendication 1, 2, 3 ou 4, dans lequel Het représente un groupe pyridyle.

6. Procédé selon la revendication 1, caractérisé en ce que l'on prépare un composé du groupe formé par les suivants:
le (E)-2-butène-dioate de 3-[7-[4-(2,2-diphényléthényl)-1-pipéridinyl]-heptyl]-pyridine (1:1) à l'état d'hémihydrate,
le (E)-2-butène-dioate de 3-[6-[4-(2,2-diphényléthényl)-1-pipéridinyl]-hexyl]-pyridine (1:1),
le (E)-2-butène-dioate de 3-[5-[4-(2,2-diphényléthényl)-1-pipéridinyl]-pentyl]-pyridine (1:1), et spécialement
la 3-[8-[4-(2,2-diphényléthényl)-1-pipéridinyl]-octyl]-pyridine, et
la 3-[10-[4-(2,2-diphényléthényl)-1-pipéridinyl]-décyl]-pyridine.